Europäisches Patentamt
European Patent Office
Office européen des brevets

(19)

(11) Publication number: **0 145 459 A2**

(12) # EUROPEAN PATENT APPLICATION

(21) Application number: **84308492.2**

(22) Date of filing: **06.12.84**

(51) Int. Cl.⁴: **C 07 D 209/16**, A 61 K 31/40

(30) Priority: **06.12.83 GB 8332434**

(43) Date of publication of application: **19.06.85 Bulletin 85/25**

(84) Designated Contracting States: **AT BE CH DE FR GB IT LI NL SE**

(71) Applicant: **GLAXO GROUP LIMITED, Clarges House 6-12 Clarges Street, London W1Y 8DH (GB)**

(72) Inventor: **Butina, Darko, 81 High Street, Arlesey Bedfordshire (GB)**

(74) Representative: **Kyle, Diana et al, ELKINGTON AND FIFE High Holborn House 52/54 High Holborn, London WC1V 6SH (GB)**

(54) **Process for the production of chemical compounds.**


(57) A process is disclosed for the preparation of an indole of general formula (I)

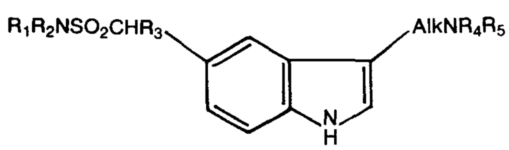

(I)

wherein $R_1$ represents H or $C_{1-6}$ alkyl or $C_{3-6}$ alkenyl,
$R_2$ represents H or $C_{1-3}$ alkyl, $C_{3-6}$ alkenyl, aryl, ar($C_{1-4}$)alkyl or $C_{5-7}$ cycloalkyl,
$R_3$ represents H or $C_{1-3}$ alkyl group;
$R_4$ and $R_5$, represent H or a $C_{1-3}$ alkyl or propenyl group or together form an aralkylidene group; and Alk represents a $C_{2-3}$ alkylene chain,
or a physiologically acceptable salt or solvate thereof which comprises reacting an indole of general formula (II):

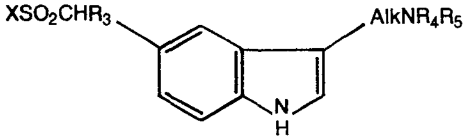

(II)

(wherein X represents a leaving group) with an amine $R_1R_2NH$.

The indoles of formula (I) are indicated for use in the treatment of migraine.


EP 0 145 459 A2

Title: PROCESS FOR THE PRODUCTION OF CHEMICAL COMPOUNDS

The present invention relates to a process for the production of indoles and, more particularly to a process for the production of indoles which are described and claimed in UK Published Patent Application No. 2124210A.

The indoles described and claimed in UK Published Patent Application No. 2124210A exhibit selective vasoconstrictor activity and are indicated for use in the treatment of migraine. Various processes for their preparation are also described in UK Published Patent Application No. 2124210A. The present invention relates to a further process for the preparation of these indoles.

Thus, the present invention provides a process for the preparation of indoles of the general formula (I):

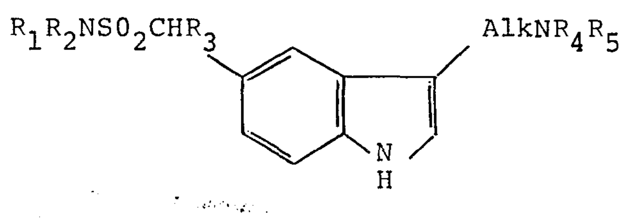

(I)

wherein

$R_1$ represents a hydrogen atom or a $C_{1-6}$ alkyl or

$C_{3-6}$ alkenyl group;

$R_2$ represents a hydrogen atom or a $C_{1-3}$ alkyl, $C_{3-6}$ alkenyl, aryl, ar$(C_{1-4})$alkyl or $C_{5-7}$ cycloalkyl group;

$R_3$ represents a hydrogen atom or a $C_{1-3}$ alkyl group;

$R_4$ and $R_5$, which may be the same or different, each represents a hydrogen atom or a $C_{1-3}$ alkyl or propenyl group or $R_4$ and $R_5$ together form an aralkylidene group; and

Alk represents an alkylene chain containing two or three carbon atoms which may be unsubstituted or substituted by not more than two $C_{1-3}$ alkyl groups,

and physiologically acceptable salts and solvates (e.g. hydrates) thereof which comprises reacting an indole of general formula (II):

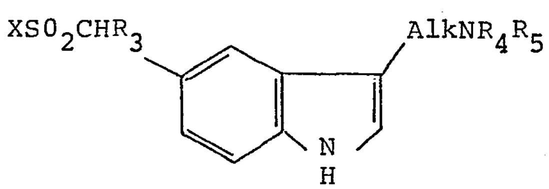

(II)

(wherein X represents a leaving group and $R_3$, $R_4$, $R_5$ and Alk are as defined for the general formula (I)) or a protected derivative thereof with a compound of general formula (III):

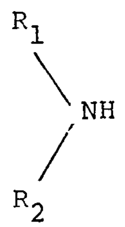

(III)

(wherein $R_1$ and $R_2$ are as defined for general formula (I)).

Examples of the leaving group X in the compounds of general formula (II) include a halogen atom (e.g. a fluorine, chlorine or bromine atom) or a group $OR_6$, where $R_6$ represents a hydrocarbyl group such as an aryl group, e.g. phenyl. The aryl moiety may be unsubstituted or substituted by one or more substituents such as halogen atoms; or nitro; cyano; amino; alkyl e.g. methyl; alkoxy e.g. methoxy; acyl, e.g. acetyl and alkoxycarbonyl e.g. ethoxycarbonyl groups. Preferably, X represents a group $OR_6$, most preferably an aryloxy group especially phenoxy.

The reaction is conveniently carried out in the presence of a solvent and may be effected in an aqueous or non-aqueous reaction medium.

The reaction medium may thus comprise one or more organic solvents, such as ethers, e.g. cyclic ethers such as dioxan or tetrahydrofuran, and acyclic ethers such as diethylether; amides e.g. N,N-dimethylformamide or N-methylpyrrolidone; alcohols e.g. methanol or ethanol;

esters e.g. ethyl acetate; nitriles e.g. acetonitrile; halogenated hydrocarbons e.g. dichloromethane; and tertiary amines e.g. triethylamine, or pyridine optionally in the presence of water. In some cases the amine of formula (III) may itself serve as the solvent.

If desired the aminolysis may be effected in the presence of a base, such as an alkali metal carbonate or bicarbonate; (e.g. sodium or potassium carbonate or bicarbonate) a tertiary amine (e.g. triethylamine or pyridine); an alkoxide (e.g. sodium t-butoxide) or a hydride (e.g. sodium hydride).

The reaction may conveniently be effected at a temperature of from -20° to +150°C.

The following reactions, in any appropriate sequence, may if necessary and/or desired be carried out subsequent to the above described process;

(i) conversion of one compound of general formula (I) or a salt or protected derivative thereof into another compound of general formula (I);

(ii) removal of any protecting groups; and

(iii) conversion of a compound of general formula (I) or a salt thereof into a physiologically acceptable salt or solvate (e.g. hydrate) thereof.

Thus, a compound of general formula (I) may be converted into another compound of general formula (I) using conventional procedures.

For example, a compound of general formula (I) wherein one or more of $R_1$, $R_2$, $R_4$ and $R_5$ are alkyl groups may be prepared from the corresponding compounds of

formula (I) wherein one or more of $R_1$, $R_2$, $R_4$ and $R_5$ represent hydrogen atoms, by reaction with a suitable alkylating agent such as a compound of formula $R_xY$ where $R_x$ represents the desired $R_1$, $R_2$, $R_4$ and $R_5$ group and Y represents a leaving group such as a halogen atom or a tosylate group; or a sulphate $(R_x)_2SO_4$. Thus the alkylating agent may be for example an alkyl halide (e.g. methyl or ethyl iodide), alkyl tosylate (e.g. methyl tosylate) or dialkylsulphate (e.g. dimethylsulphate). The alkylation reaction may be conveniently carried out in an inert organic solvent such as an amide (e.g. dimethylformamide), an ether (e.g. tetrahydrofuran) or an aromatic hydrocarbon (e.g. toluene) preferably in the presence of a base. Suitable bases include, for example, alkali metal hydrides, such as sodium hydride; alkali metal amides, such as sodium amide; alkali metal carbonates, such as sodium carbonate; and alkali metal alkoxides such as sodium or potassium methoxide, ethoxide or t-butoxide.

Compounds of formula (I) wherein $R_1$ represents an alkenyl group, $R_2$ represents an alkenyl, aralkyl, or cycloalkyl group and/or one or both of $R_4$ and $R_5$ represents propenyl, may be prepared similarly, using an appropriate compound of formula $R_xY$ or $(R_x)_2SO_4$.

The starting materials of general formula (II)

0145459



wherein X represents a group $OR_6$ may be prepared, for example by reduction of a compound of general formula (IV):

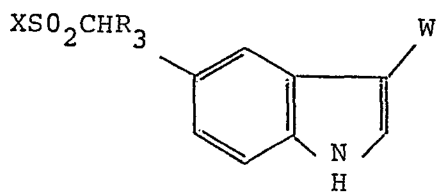

(IV)

(wherein X and $R_3$ are as previously defined above and W is a group capable of being reduced to give the required $AlkNR_4R_5$ group or to give a protected derivative of $AlkNR_4R_5$),

or a salt or protected derivative thereof.

Examples of the group W include the group $TNO_2$ (where T is Alk or an alkenyl group corresponding to the group Alk); $(CHR_7)_{\underline{x}}CHR_8CN$; $AlkNR_4COR_5'$ and $-COCONR_4R_5$ (where $R_7$ and $R_8$ which may be the same or different each represents a hydrogen atom or a $C_{1-3}$ alkyl group, $\underline{x}$ is zero or 1, and $R_5'$ is part of the group $R_5$ or the group $OR_{\underline{c}}$ where $R_{\underline{c}}$ is an alkyl or aralkyl group).

Suitable reducing agents include hydrogen in the presence of a metal catalyst (e.g. rhodium on alumina or palladium on charcoal); alkali metal or alkaline earth metal borohydrides or cyanoborohydrides (e.g. sodium or calcium borohydride); and metal hydrides such as lithium aluminium hydride.

Examples of the group W and methods for its reduction are described in more detail in UK Published Patent Application No. 2124210A.

A compound of formula (II) wherein X represents a halogen atom may be prepared, for example by reacting the corresponding sulphonic acid derivative or a salt thereof with a halogenating agent such as a phosphorus halide or oxyhalide in an inert organic solvent e.g. phosphorus pentachloride in dichloromethane.

A sulphonic acid of formula (II) may be prepared for example by acid or base catalysed hydrolysis of an ester of formula (II), i.e. a compound wherein X represents the group $OR_6$.

A compound of general formula (II) where X preferably does not represent a halogen atom, may also be prepared by cyclisation of a compound of general formula (V):

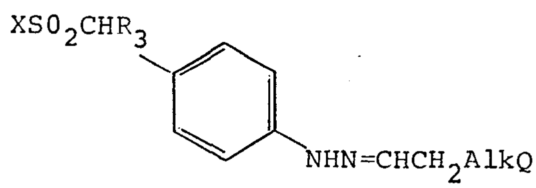

(V)

(where Q is the group $NR_4R_5$ or a protected derivative thereof or a leaving group such as a halogen atom (e.g. chlorine or bromine) or an acyloxy group such as a carboxylic or sulphonic acyloxy group (e.g. an acetoxy, dichloroacetoxy, p-toluenesulphonyloxy or methanesulphonyloxy group)).

The reaction may be effected in an aqueous organic

reaction medium, such as an aqueous alcohol or aqueous ether. Where Q is the group $NR_4R_5$ (or a protected derivative thereof) the reaction is desirably effected in the presence of an acid catalyst. This general method is described in more detail in UK Published Patent Application No. 2124210A.

Compounds of general formulae (IV) and (V) may be prepared by analogous methods to those described in UK Published Patent Application No. 2035310 and "A Chemistry of Heterocyclic Compounds - Indoles Part II" Chapter VI edited by W J Hamilton (1972) Wiley Interscience, New York, as well as UK Published Patent Application No. 2124210A.

Compounds of general formulae (II), (IV) and (V) as previously defined and also sulphonic acid of formula (II) (wherein X is a hydrogen atom) and salts thereof, are novel compounds and constitute a further aspect of the present invention.

Compounds of general formula (II) exhibit potent and selective vasoconstrictor activity as described for compounds of UK Published Patent Application No. 2124210A. Thus compounds of general formula (II) selectively constrict the carotid arterial bed of the anaesthetised dog, whilst having a negligible effect on blood pressure. The selective vasoconstrictor action of compounds (II) has been demonstrated _in vitro_.

It should be appreciated that in some of the above transformations it may be necessary or desirable to protect any sensitive groups in the molecule of the compound in question to avoid undesirable side reactions. For example, during any of the reaction sequences described above, it may be necessary to protect the group $NR_4R_5$, wherein $R_4$ and/or $R_5$ represents hydrogen, by protonation or with a group easily removable at the end of the

reaction sequence. Such groups may include, for example, aralkyl groups, such as benzyl, diphenylmethyl or triphenylmethyl or acyl groups such as N-benzyloxycarbonyl or t-butoxycarbonyl or phthaloyl.

In some cases, it may also be desirable to protect the indole nitrogen with, for example, an aralkyl group such as benzyl.

Subsequent cleavage of the protecting group may be achieved by conventional procedures. Thus an aralkyl group such as benzyl, may be cleaved by hydrogenolysis in the presence of a catalyst (eg. palladium on charcoal) or sodium and liquid ammonia; an acyl group such as N-benzyloxycarbonyl may be removed by hydrolysis with, for example, hydrogen bromide in acetic acid or by reduction, for example by catalytic hydrogenation. The phthaloyl group may be removed by hydrazinolysis (e.g. by treatment with hydrazine hydrate) or by treatment with a primary amine (e.g. methylamine).

Where it is desired to isolate a compound of formula (I) as a physiologically acceptable salt, for example as an acid addition salt, this may be achieved by treating the free base of general formula (I), with an appropriate acid (e.g. succinic or hydrochloric acid) preferably with an equivalent amount in a suitable solvent (e.g. aqueous ethanol).

Referring to the general formula (I), the alkyl

groups in the general formula (I) may be straight chain or branched chain alkyl groups containing 1 to 3 carbon atoms, or in the case of $R_1$, 1 to 6, preferably 1 to 3, carbon atoms. Examples of an alkyl group include methyl, ethyl, propyl and isopropyl groups. The alkenyl groups preferably contain 3 or 4 carbon atoms, examples of which include propenyl and butenyl groups. The cycloalkyl groups preferably contain 5 or 6 carbon atoms and examples include cyclopentyl and cyclohexyl groups. The term aryl, used as such or in the term aralkyl, preferably means phenyl. The alkyl moieties of the aralkyl groups preferably contain 1 or 2 carbon atoms. Examples of an aralkyl group include benzyl and phenethyl groups. The aralkylidene group is preferably an arylmethylidene group such as benzylidene.

Suitable physiologically acceptable salts of the indoles of general formula (I) include acid addition salts formed with organic or inorganic acid for example hydrochlorides, hydrobromides, sulphates, fumarates, maleates and succinates. Other salts may be useful in the preparation of the compounds of general formula (I) e.g. creatinine sulphate adducts.

A preferred class of compounds represented by the

general formula (I) is that in which $R_1$ represents a hydrogen atom or a $C_{1-6}$ alkyl group and $R_2$ represents a hydrogen atom or a $C_{1-3}$ alkyl or ar($C_{1-4}$)alkyl group.

Another preferred class of compounds represented by the general formula (I) is that in which $R_3$, represents a hydrogen atom.

A further preferred class of compounds is that wherein, in the general formula (I), $R_4$ and $R_5$, which may be the same or different, each represents a hydrogen atom or a $C_{1-3}$ alkyl group, for example, a methyl group.

A preferred class of compounds falling within the scope of general formula (I) is that wherein $R_1$ represents a hydrogen atom or a $C_{1-3}$ alkyl group e.g. a methyl group; $R_2$ represents a hydrogen atom or a $C_{1-3}$ alkyl group, e.g. a methyl, ethyl or isopropyl group, or an ar($C_{1-2}$)alkyl group e.g. a benzyl group; $R_3$ represents a hydrogen atom; and $R_4$ and $R_5$, which may be the same or different, each represents a hydrogen atom or a $C_{1-3}$ alkyl group e.g. a methyl group; and physiologically acceptable salts and solvates (e.g. hydrates) thereof.

The invention is further illustrated by the following Examples in which temperatures are in °C.

The invention is further illustrated by the following examples. All temperatures are in $^0C$. 'Hyflo' is a filtration aid. Reactivials are 4ml stout-walled glass vials with a screw cap and teflon-faced disc, supplied by Pierce and Warriner (UK Ltd. Chromatography was carried out either in the conventional manner using silica gel (Merck, Kieselgel 60, Art. 7734) and thin layer chromatography (t.l.c) on silica (Macherly-Nagel, Polygram) except where otherwise stated. The following abbreviations define the eluent used for chromatography and t.l.c.

(A) Methylene chloride-ethanol-0.88 ammonia 50:8:1

(B) Methylene chloride-ethanol-0.88 ammonia 20:8:1

(C) Methylene chloride-ethanol-0.88 ammonia 100:8:1

(D) Ethyl acetate-isopropanol-water-0.88 ammonia 25:15:8:1

Intermediates were routinely checked for purity by t.l.c. employing u.v. light for detection and spray reagents such as potassium permanganate ($KMnO_4$). In addition indolic intermediates were detected by spraying with aqueous ceric sulphate ($Ce^{IV}$) and tryptamines by spraying with a solution of iodoplatinic acid (IPA) or ceric sulphate.

Proton ($^1H$) nuclear magnetic resonance (n.m.r.) spectra were obtained either at 90MHz using a Varian EM390 instrument or at 250MHz using a Bruker AM or WM250 instrument. s = singlet, m = multiplet.

Example 1

3-(2-Aminoethyl)-N-methyl-1H-indole-5-methanesulphonamide

(i) Phenyl 4-aminobenzenemethanesulphonate hydrochloride

A solution of phenyl 4-nitrobenzenemethanesulphonate (1.6g) in ethyl acetate (200ml) and concentrated hydrochloric acid (1ml) was hydrogenated over prereduced 5% palladium oxide on charcoal (50% aqueous paste 0.4g) in ethanol (10ml) at atmospheric pressure and temperature. The catalyst was filtered off and the filtrate concentrated to give the title compound as a solid (1.55g). A small sample (0.2g) was crystallised from isopropyl acetate (10ml) and a few drops of methanol to give analytically pure material as a solid (70mg) m.p. 147-148$^0$.

(ii) Phenyl 4-hydrazinobenzenemethanesulphonate hydrochloride

To a thick suspension of the product of Stage (i) (1.5g) in water (10ml) and conc. hydrochloric acid (10ml) was added a solution of sodium nitrite (0.35g) in water (2ml) keeping the temperature below $-2^0$. More water (5ml) was added to the resulting suspension and the cloudy solution added to a solution of tin (II) chloride dihydrate (5.6g) in conc. hydrochloric acid (10ml) keeping the temperature below $-2^0$. The resulting suspension was stirred at room temperature for 1h, then the precipitate was filtered off, washed with ether (200ml) and dried in vacuo ($45^0$; 24h) to give a white solid (1g). A sample (0.1g) was crystallised from isopropanol (10ml)/methanol (2ml) to give the title compound as a powder (30mg) m.p. $148-149^0$.

(iii) Phenyl 4-[2-(3-cyano-1-propylidene)hydrazino]benzenemethanesulphonate, compound with water (40:1)

A solution of the product of Stage (ii) (1g) and 3-cyanopropanal dimethylacetal (0.5g) in water (20ml) was treated with dilute hydrochloric acid (2N; 5 drops) and stirred for 16h at room temperature. The resulting solid was filtered off, washed with water (20ml), ether (20ml) and dried in vacuo at $20^0$ for 2h to give the title compound (0.9g) m.p. $95-96^0$.

(iv) Phenyl 3-(cyanomethyl)-1H-indole-5-methanesulphonate

A suspension of the product of Stage (iii) (0.8g) in polyphosphate ester (8g) and chloroform (16ml) was heated at reflux for 5 minutes, and then poured onto ice (50g). The resulting suspension was stirred with ice for 20 minutes, then extracted with chloroform (3x20ml). The organic extracts were washed with sodium bicarbonate (8%; 2x20ml), water (20ml) and dried ($MgSO_4$). The crude product was purified by flash chromatography (3cm; Merck 9835) eluting with ether and the nitrile obtained as a solid which was triturated with ether (1ml) and dried in vacuo at $20^0$ to give the title compound (0.27g) m.p. $134-135^0$.

(v) Phenyl 3-(2-aminoethyl)-1H-indole-5-methanesulphonate

A solution of the product of Stage (iv) (163mg) in ethanol (15ml) and conc. hydrochloric acid (0.15ml) was hydrogenated at atmospheric

pressure over 5% rhodium on alumina for 19h. The resulting mixture was filtered, diluted with water (150ml), acidified to pH 1 with 2N hydrochloric acid and washed with diethyl ether. The aqueous layer was basified with excess solid potassium carbonate and extracted with ethyl acetate. Evaporation of the ethyl acetate extracts gave the title compound (50mg) as a gum.

N.m.r. $\delta(CD_3OD)$: 7.53(1H,m,indole-4); 7.30-7.00(8H,m,aromatics); 4.60(2H,s,$PhO_3SCH_2$); 2.78(4H,brs,$CH_2CH_2NH_2$).

T.l.c.: (A) Rf 0.24.

(vi) 3-(2-Aminoethyl)-N-methyl-1H-indole-5-methanesulphonamide

A solution of the product of Stage (v) (30mg) in 33% w/v ethanolic methylamine was heated at 110$^0$ for 2h in a sealed container. The resulting solution was evaporated to afford the title compound as a gum (30mg).

T.l.c. (B) Rf 0.28 identical with that of an authentic sample, (prepared as in Published UK Patent Application No.2124210A, Example 1c) which exhibited the following n.m.r. spectral characteristics: $\delta(CD_3SOCD_3)$2.59(3H,s,$CH_3NHSO_2$); 2.75-2.95 (4H,m,$CH_2CH_2NH_2$); 4.38(2H,s,$CH_2SO_2$) 7.1-7.6(4H,m,aromatic).

Example 2

3-(2-Aminoethyl)-N,N-dimethyl-1H-indole-5-methanesulphonamide

(i) Phenyl 3-(2-Aminoethyl)-1H-indole-5-methanesulphonate,oxalate

A hot solution the product of Example 1 Stage (v) (0.18g) in ethanol (5ml) was treated with oxalic acid (50mg) and the salt precipitated at once. More ethanol (5ml) was added, the hot solution filtered and the filtrate on cooling produced the title compound as a solid (0.16g) m.p. 162-163$^0$.

Analysis Found: C,54.1;H,7.9;N,6.5.

$C_{17}H_{18}N_2O_3S.C_2H_2O_4.0.2H_2O$ requires C,53.8;H,7.85;N,6.5%.

T.l.c. (C) Rf 0.15 ($Ce^{IV}$IPA).

(ii) 3-(2-Aminoethyl)-N,N-dimethyl-1H-indole-5-methanesulphonamide

A solution of the product of Stage (i) as the free base (0.18g) in pyridine (2ml) and dimethylamine (2ml) was heated at 100$^0$ in a reactivial overnight. All the solvent was evaporated and the residue purified by column chromatography (A) to give the title compound as an

oil (55mg) which was shown by t.l.c. (B, Rf 0.35) to be identical to an authentic sample (prepared as in Published UK Patent Application No. 2124210A, Example 12c).

N.m.r. δ($CD_3SOCD_3$)2.70(6H,s,$Me_2NSO_2$); 3.04(4H,brs,$CH_2CH_2NH_2$); 4.43(2H,s,$SO_2CH_2$); 7.1-7.65(4H,m,aromatic).

Example 3

3-(2-Aminoethyl)-N-(phenylmethyl)-1H-indole-5-methanesulphonamide

A solution of the product of Example 1 Stage (v) (0.27g) in pyridine (2ml) and benzylamine (2ml) was heated at $100^0$ in a 'reactivial' overnight. Most of the pyridine was removed under reduced pressure, the residue washed with ether and then purified by chromatography (A) to give the title compound as an oil (91mg) which was shown by t.l.c. (D, Rf 0.4) to be identical with an authentic sample (prepared as in Published UK Patent Application No.2124210A, Example 9d).

N.m.r. δ($CD_3SOCD_3$)2.8-3.0(4H,m,$CH_2CH_2NH_2$); 4.10(2H,s,$CH_2NHSO_2$); 4.38(2H,s,$NHSO_2CH_2$); 7.1-7.6(10H,m,aromatic +$NHSO_2$).

Example 4

3-(2-Aminoethyl)-N-(2-propenyl)-1H-indole-5-methanesulphonamide

A solution of the product of Example 1 Stage (v) (0.2g) in pyridine (2ml) and allylamine (2ml) was heated at $100^0$ in a 'reactivial' overnight. All the solvent was evaporated and the residue purified by column chromatography (B) to give the title compound as an oil (0.1g) which was shown by t.l.c. (B, Rf 0.3) to be identical with an authentic sample (prepared as in Published UK Patent Application No. 2124210A, Example 14d).

N.m.r. δ($CD_3SOCD_3$)2.8-3.0(4H,m,$CH_2CH_2NH_2$), 3.5(2H,m,$CH_2NHSO_2$); 4.38(2H,s,$NHSO_2CH_2$); 5.05-5.3(2H,m,$CH_2$=$CHCH_2$); 5.7-5.9(1H,m,$CHCH_2$); 7.1-7.6(4H,m,aromatic).

Example 5

3-[2-(Dimethylamino)ethyl]-1H-indole-5-methanesulphonamide

(i) Phenyl 3-[2-(Dimethylamino)ethyl]-1H-indole-5-methanesulphonate

4,4-Dimethoxy-N,N-dimethylbutanamine (5.64g) and the product of Example 1 Stage (ii) (10g) in water (200ml) containing aqueous 2N

hydrochloric acid (17.1ml) were stirred at $26^0$ for 1.5h. Chloroform (100ml) was added and the pH was adjusted to 10 with aqueous sodium carbonate (2N). After separation, the aqueous layer was further extracted with chloroform (1x50ml, 1x30ml). The combined chloroform extracts were dried ($Na_2SO_4$) and added to polyphosphate ester (50g) in chloroform (30ml). The mixture was stirred at $26^0$ for 0.5h then at gentle reflux for 1h. Aqueous sodium carbonate (8%, 100ml) was added, the layers separated and the organic layer evaporated to dryness at reduced pressure to give an oil (17.8g). The oil was purified by column chromatography (C) to give the title compound as a solid, (2.6g) m.p. $124-126^0$.

T.l.c. (C), Rf 0.5 (IPA)

(ii) 3-[2-(Dimethylamino)ethyl]-1H-indole-5-methanesulphonamide

A solution of the product of Stage (i) (0.1g) in a solution of ammonia in pyridine (4ml) (prepared by adding liquid ammonia (3ml) to pyridine (15ml) with cooling) was heated at $100^0$ in a 'reactivial' for 16h. Solvent was evaporated and the residue purified by column chromatography (A) to give the title compound as an oil (57mg) which was shown by and t.l.c. (B, Rf 0.35) to be identical to an authentic sample (prepared as in Published UK Patent Application No.2124210A, Example 21).

N.m.r δ($CDCl_3/CD_3OD$) 2.3(6H,s,$NMe_2$); 2.4-3.0(4H,m,$CH_2CH_2N$); 4.35(2H,s,$SO_2CH_2$) 6.95-7.55(4H,m,aromatic).

CLAIMS

1. A process for the preparation of an indole of general formula (I)

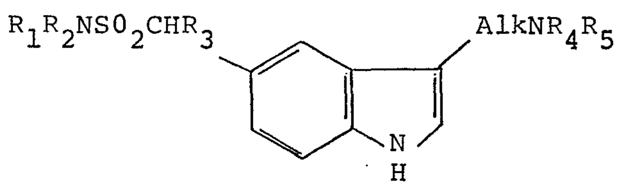

(I)

wherein

$R_1$ represents a hydrogen atom or a $C_{1-6}$ alkyl or $C_{3-6}$ alkenyl group;

$R_2$ represents a hydrogen atom or a $C_{1-3}$ alkyl, $C_{3-6}$ alkenyl, aryl, ar($C_{1-4}$)alkyl or $C_{5-7}$ cycloalkyl group;

$R_3$ represents a hydrogen atom or a $C_{1-3}$ alkyl group;

$R_4$ and $R_5$, which may be the same or different, each represents a hydrogen atom or a $C_{1-3}$ alkyl or propenyl group or $R_4$ and $R_5$ together form an aralkylidene group; and

Alk represents an alkylene chain containing two or three carbon atoms which may be unsubstituted or substituted by not more than two $C_{1-3}$ alkyl groups,

or a physiologically acceptable salt or solvate thereof which comprises

(A) reacting an indole of general formula (II):

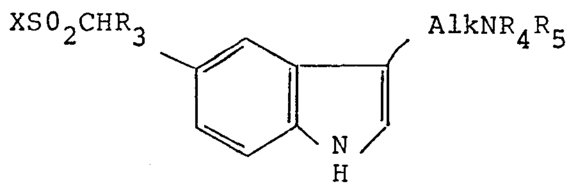

(II)

(wherein X represents a leaving group and $R_3$ and $R_4$, $R_5$ and Alk are as defined for the general formula (I)) with a compound of general formula (III):

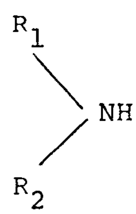

(III)

(wherein $R_1$ and $R_2$ are as defined for general formula (I));

and if necessary and/or desired subjecting the resulting compound to one or more further reactions comprising

(B) (i) converting one compound of general formula (I) or a salt or protected derivative thereof into another compound of general formula (I):

(ii) removing any protecting group or groups; and

(iii) converting a compound of general formula (I) or a salt thereof into a physiologically acceptable salt or solvate thereof.

2. A process according to claim 1, wherein in the general formula (II), X represents a halogen atom or a group $OR_6$, where $R_6$ represents a hydrocarbyl group.

3. A process according to claim 2, wherein in the general formula (II), X represents a group $OR_6$ where $R_6$ represents an aryl group which may be unsubstituted or substituted by one or more of halogen atoms and nitro cyano, amino, alkyl, alkoxy and acyl groups.

4. A process according to claim 3, wherein, in the general formula (II), X represents a phenoxy group.

5. A process according to any of claims 1 to 4, wherein the reaction is effected in a solvent.

6. A process according to any of claims 1 to 5, wherein the reaction of step (A) is effected in the presence of a base.

7. A process according to any of claims 1 to 6, wherein the reaction of step (A) is effected at a temperature of from -20° to +150°C.

8. A process according to any of claims 1 to 7, wherein step (B)(i) comprises, in order to prepare a compound of general formula (I) wherein one or more of $R_1$, $R_2$, $R_4$ and $R_5$ are alkyl groups, reacting a compound of general formula (I) wherein one or more of $R_1$, $R_2$, $R_4$ and $R_5$ represent hydrogen atoms with an alkylating agent of formula $(R_x)_2SO_4$ or $R_xY$ where $R_x$ represents the desired $R_1$, $R_2$, $R_4$ or $R_5$ group and Y represents

a leaving group.

9. A process according to any of claims 1 to 7, wherein step B(i) comprises, in order to prepare a compound of general formula (I) wherein $R_1$ represents an alkenyl group, $R_2$ represents an alkenyl, aralkyl or cycloalkyl group and/or one or both of $R_4$ and $R_5$ represents propenyl, reacting a compound of general formula (I) wherein one or more of $R_1$, $R_2$, $R_4$ and $R_5$ represent hydrogen atoms with an alkylating agent of fromula $R_xY$ or $(R_x)_2SO_4$ where $R_x$ and Y are as defined in claim 8.

10. A compound of general formula (II):

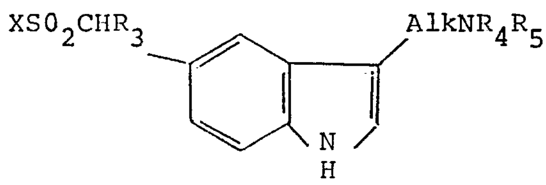

(II)

wherein $R_3$ represents a hydrogen atom or a $C_{1-3}$ alkyl group;

$R_4$ and $R_5$, which may be the same or different, each represents a hydrogen atom or a $C_{1-3}$ alkyl or propenyl group or $R_4$ and $R_5$ together form an aralkylidene group; and

X represents a hydrogen atom or a leaving group,

and salts thereof.